# EUROPEAN PATENT APPLICATION

(11) **EP 4 488 258 A1**
(43) Date of publication of application: **08.01.2025**
(21) Application number: 24177659.0
(22) Date of filing: 23.05.2024
(51) Int. Cl.: C07C 67/54, C07C 69/54

(54) **IMPROVED DISTILLATION METHOD FOR PURIFICATION OF ACRYLIC AND VINYL MONOMERS**

(71) Applicant: Evonik Operations GmbH, 45128 Essen (DE)
(72) Inventor: JAMES, Dr., Philip, SA70 7LE Tenby (GB); GÄRTNER, Dr., Felix, 45721 Haltern am See (DE)
(74) Representative: Evonik Patent Association

(57) **Abstract**

The present invention relates to an improved distillation method for purification of certain acrylic and vinyl monomers **M,** preferably acrylic monomers, using particularly advantageous polymerization inhibitors I, which are derivatives of 2,2,6,6-tetramethyl piperidine-*N*-oxyl. It further relates to mixtures **M₁** comprising inhibitor I and monomer **M** and the use of the inhibitor **I** to inhibit polymerization of a monomer **M** during the purification **of M** by distillation.

## Description

The present invention relates to an improved distillation method for purification of certain acrylic and vinyl monomers **M**, preferably acrylic monomers, using particularly advantageous polymerization inhibitors **I**, which are derivatives of 2,2,6,6-tetramethyl piperidine-*N*-oxyl. It further relates to mixtures **M₁** comprising inhibitor **I** and monomer **M** and the use of the inhibitor **I** to inhibit polymerization of a monomer **M** during the purification of **M** by distillation.

### Background of the Invention

Certain unsaturated compounds such as vinyl monomers (for example, styrene and its derivatives) or acrylic monomers [for example, (meth)acrylic acid and its derivatives] tend to polymerize due to heating, light, etc.

This property is disadvantageous during production and storage of these unsaturated compounds, and in particular poses a technological challenge when these compounds are purified by distillation.

To inhibit the polymerization of these compounds, inhibitors are conventionally added to the monomer. A particular class of inhibitors used in this context are so-called TEMPO-molecules, i.e. 2,2,6,6-tetramethyl piperidine-*N*-oxyl and its derivatives. "TEMPO" is an abbreviation for "2,2,6,6-tetramethyl piperidine-*N-*oxyl".

For example, JP 2006-169220 A discloses addition of 4-acetamido-TEMPO during esterification of acrylic acid with alcohols containing ether groups (ethoxyethanol, tetrahydrofurfuryl alcohol).

WO 2018/234774 A1 discloses different TEMPO derivatives that are suitable as inhibitors for different monomers, and in particular discloses the use of 4-hydroxy-2,2,6,6-tetramethyl piperidine-*N*-oxyl ("4-OH-TEMPO") to inhibit the polymerization of methyl methacrylate.

CN 116573985 A discloses different TEMPO derivatives for inhibition of monomer polymerization, in particular methyl methacrylate, among those 4-acetamido-TEMPO.

While TEMPO derivatives thus have proven to be effective in inhibiting polymerization of industrially important monomers, it was now observed that, nevertheless, the application of TEMPOs during distillation of certain monomers might lead to problems. Namely, during distillation of certain monomers, it was observed that the TEMPO derivative is co-distilled with the monomer and then found in the distillation fraction of the monomer. This problem was observed in particular for certain alkyl derivatives of (meth)acrylic acid as well as derivatives of styrene when TEMPO derivatives such as 4-OH-TEMPO or 4-acetoxy-2,2,6,6-tetramethylpiperidine-*N*-oxyl (abbreviated herein as "4-Acetoxy-TEMPO") were used as inhibitor during distillation.

The problem underlying the present invention hence was to provide a TEMPO inhibitor that would efficiently inhibit the polymerization of these alkyl derivatives and yet not display the above disadvantage of being co-distilled with the monomer.

### Figure

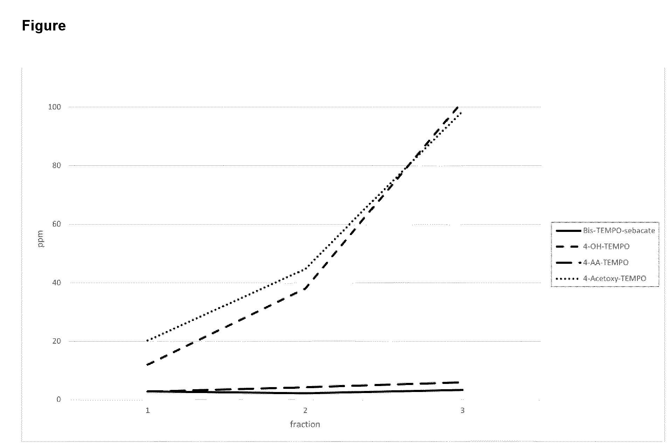

The Figure shows the content of inhibitor molecules found in three fractions after distillation of 2-ethylhexyl acrylate. The x-axis gives the respective fraction (fraction 1 is taken until 15 minutes after start of the distillation, fraction 2 is taken from 15 to 215 minutes after start of the distillation, fraction 3 is taken from 215 to 275 minutes after start of the distillation).

The y-axis gives the content of the respective inhibitor in the fraction of distilled 2-ethylhexyl acrylate (in ppm).

Bis-TEMPO-sebacate, 4-AA-TEMPO are inhibitors according to the invention.

4-OH-TEMPO, 4-Acetoxy-TEMPO are comparative inhibitors.

### Detailed Description of the Invention

It was now surprisingly found that this problem is solved by the method of purifying according to the present invention, wherein a certain inhibitor **I** is applied. A mixture **M₁** comprising the inhibitor **I** and the use of at least one inhibitor **I** for inhibiting the polymerization of certain monomers **M** have, in further aspects of the invention, also proven to be advantageous.

Namely, it was found that pure fractions of certain acrylic or vinylic monomers **M**, and in particular 2-ethylhexyl acrylate, can be obtained when, during distillation, certain inhibitors **I** are used, to inhibit the polymerization of these monomers **M.** These inhibitors **I** are TEMPO inhibitors, most preferably **I** = 4-acetamido TEMPO. This was even more surprising as the tendency of the inhibitors according to the invention to stay in the sump (and thus to **not** co-distill) did not merely correlate with their boiling point. The inhibitors **I** according to the invention rather showed a quantitative and qualitative improved tendency to stay in the sump and not co-distill, compared to other TEMPO derivatives such as 4-OH-TEMPO and 4-acetoxy-TEMPO.

### 1. Method of purifying a monomer M

In a first aspect the present invention hence relates to a method of purifying a monomer **M** by distillation. The monomer **M** is selected from compounds according to chemical formulae **(A)**, **(B).** Preferably, the monomer **M** is selected from compounds according to chemical formula **(A).**

Chemical formula **(A)** has the following structure:

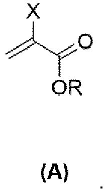

In formula **(A)**, X is selected from the group consisting of hydrogen, methyl, and is preferably hydrogen.

In formula **(A)**, R is selected from C₂ to C₂₀ alkyl, C₂ to C₂₀ hydroxyalkyl, preferably R is selected from C₄ to C₂₀ alkyl, C₂ to C₂₀ hydroxyalkyl, more preferably R is selected from C₅ to C₁₅ alkyl, C₂ to C₁₅ hydroxyalkyl, even more preferably R is C₅ to C₁₅ alkyl, even more preferably R is C₅ to C₁₂ alkyl, most preferably R = 2-ethylhexyl. Hence, most preferably, the compound according to chemical formula **(A)** is 2-ethylhexyl-acrylate (CAS-No.: 103-11-7).

"Alkyl" may be linear or branched alkyl.

"Hydroxyalkyl" is an alkyl group wherein at least one hydrogen is substituted by an OH group. Preferably, a hydroxyalkyl carries one to four hydroxy groups. More preferably, a hydroxyalkyl group carries one or two hydroxy groups. Most preferably, a hydroxyalkyl group carries one hydroxy group.

C₂ to C₂₀ hydroxyalkyl and C₂ to C₁₅ hydroxyalkyl are each preferably selected from 1-hydroxy-ethyl, 2-hydroxy-ethyl. C₂ to C₂₀ hydroxyalkyl and C₂ to C₁₅ hydroxyalkyl are each most preferably selected from 2-hydroxy-ethyl.

Chemical formula **(B)** has the following structure:

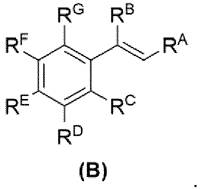

In formula **(B)**, at least one of R^{A}, R^{B}, R^{C}, R^{D}, R^{E}, R^{F}, R^{G} is selected from C₁ to C₂₀ alkyl, C₂ to C₂₀ alkenyl, and each of the other residues R^{A}, R^{B}, R^{C}, R^{D}, R^{E}, R^{F}, R^{G} is hydrogen. Preferably, in formula **(B)**, one of R^{A}, R^{B}, R^{C}, R^{D}, R^{E}, R^{F}, R^{G} is selected from C₁ to C₂₀ alkyl, C₂ to C₂₀ alkenyl, and each of the other residues R^{A}, R^{B}, R^{C}, R^{D}, R^{E}, R^{F}, R^{G} is hydrogen.

In a preferred embodiment of formula **(B)**, at least one of R^{A}, R^{B}, R^{C}, R^{D}, R^{E}, R^{F}, R^{G} is selected from C₁ to C₆ alkyl, C₂ to C₆ alkenyl, and each of the other residues R^{A}, R^{B}, R^{C}, R^{D}, R^{E}, R^{F}, R^{G} is hydrogen. Preferably, in formula **(B)**, one of R^{A}, R^{B}, R^{C}, R^{D}, R^{E}, R^{F}, R^{G} is selected from C₁ to C₆ alkyl, C₂ to C₆ alkenyl, and each of the other residues R^{A}, R^{B}, R^{C}, R^{D}, R^{E}, R^{F}, R^{G} is hydrogen.

In an even more preferred embodiment of formula **(B)**, at least one of R^{A}, R^{B}, R^{C}, R^{D}, R^{E}, R^{F}, R^{G} is selected from C₁ to C₄ alkyl, C₂ to C₄ alkenyl, and each of the other residues R^{A}, R^{B}, R^{C}, R^{D}, R^{E}, R^{F}, R^{G} is hydrogen. Preferably, in formula **(B)**, one of R^{A}, R^{B}, R^{C}, R^{D}, R^{E}, R^{F}, R^{G} is selected from C₁ to C₄ alkyl, C₂ to C₄ alkenyl, and each of the other residues R^{A}, R^{B}, R^{C}, R^{D}, R^{E}, R^{F}, R^{G} is hydrogen.

The most preferred compounds according to formula **(B)** are selected from
- α-methyl styrene (R^{B} = methyl; R^{A} = R^{C} = R^{D} = R^{E} = R^{F} = R^{G} = H);
- 1,2-divinyl benzene (R^{C} = -(CH)=CH₂; R^{A} = R^{B} = R^{D} = R^{E} = R^{F} = R^{G} = H);
- 1,3-divinyl benzene (R^{D} = -(CH)=CH₂; R^{A} = R^{B} = R^{C} = R^{E} = R^{F} = R^{G} = H);
- 1,4-divinyl benzene (R^{E} = -(CH)=CH₂; R^{A} = R^{B} = R^{C} = R^{D} = R^{F} = R^{G} = H);
- 2-tert-butyl styrene (R^{C} = tert-butyl; R^{A} = R^{B} = R^{D} = R^{E} = R^{F} = R^{G} = H);
- 3-tert-butyl styrene (R^{D} = tert-butyl; R^{A} = R^{B} = R^{C} = R^{E} = R^{F} = R^{G} = H);
- 4-tert-butyl styrene (R^{E} = tert-butyl; R^{A} = R^{B} = R^{G} = R^{D} = R^{F} = R^{G} = H);
- 2-vinyl toluene (R^{C} = -CH₃; R^{A} = R^{B} = R^{D} = R^{E} = R^{F} = R^{G} = H);
- 3-vinyl toluene (R^{D} = -CH₃; R^{A} = R^{B} = R^{C} = R^{E} = R^{F} = R^{G} = H);
- 4-vinyl toluene (R^{E} = -CH₃; R^{A} = R^{B} = R^{G} = R^{D} = R^{F} = R^{G} = H).

The most preferred monomer **M is** 2-ethylhexyl acrylate (CAS-Nr.: 103-11-7).

In step a. of the method according to the present invention, a liquid phase **P_{M1}** comprising **M** and at least one inhibitor **I** is provided.

The inhibitor **I** is selected from the compounds according to chemical formulae **(I), (II), (III)**, preferably is a compound according to chemical formula **(I)** or **(II),** more preferably is a compound according to chemical formula **(I):**

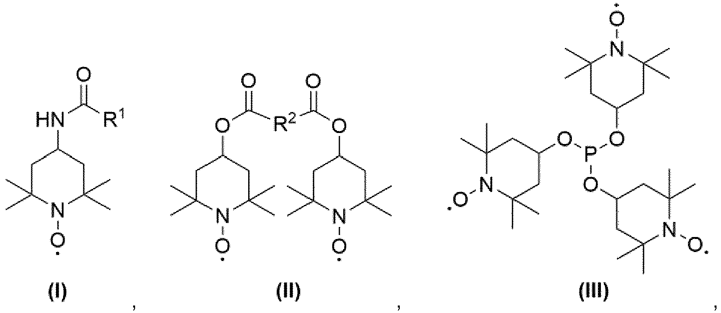

In formula **(I)**, R¹ is selected from alkyl group, alkenyl group, alkynyl group, aromatic group, preferably R¹ = alkyl group, more preferably R¹ = C₁-C₂₀ alkyl group, even more preferably R¹ = C₁-C₁₅ alkyl group, even more preferably R¹ = C₁-C₁₀ alkyl group, even more preferably R¹ = C₁-C₆ alkyl group, even more preferably R¹ = C₁-C₄ alkyl group, even more preferably R¹ = C₁-C₂ alkyl group, most preferably R¹ = methyl.

In formula **(II),** R² is a divalent group selected from alkylene group, alkenylene, alkynylene, bivalent aromatic group, preferably R² = alkylene group, more preferably R² = C₁-C₂₀ alkylene group, more preferably R² = C₄-C₁₅ alkylene group, more preferably R² = C₆-C₁₂ alkylene group, more preferably R² = C₈-C₁₀ alkylene group, most preferably R² = n-octylene group.

For R² = n-octylene group, the compound according to formula **(II)** is Bis(2,2,6,6-tetramethyl-1-piperidinyloxy-4-yl) sebacate ("Bis-TEMPO-sebacate"; CAS-No.: 2516-92-9).

The liquid phase **P_{M1}** constitutes the phase from which at least a part of **M** is purified by distillation in the method of the present invention. The liquid phase **P_{M1}** is not in any way limited and may be obtained from a raw product of a production process for **M.** This raw product may then be mixed with **I**, and then the resulting mixture comprising **M** and **I** is provided in step a. and subjected to purification by distillation according to the following steps b. and c. of the method of the invention.

The liquid phase **P_{M1}** may also be a stored product **M** which has been stored in mixture with **I** for a certain amount of time (preferably more than 5 minutes, more preferably more than 1 hour, more preferably more than a day, even more preferably more than a week or even more than a month or more than a year) and is to be purified before further use.

In step b. of the method according to the invention, the liquid phase **P_{M1}** is heated to obtain a gaseous stream **G_{M}** comprising the monomer **M.** The skilled person is aware of methods to carry out such a heating. Typically, the liquid phase **P_{M1}** is heated in step b. so that the boiling point of the monomer **M** is reached. Preferably, the monomer **M** is a compound according to chemical formulae **(A)**, **(B)** as described above and suffices the additional requirement that it has a boiling point at atmospheric pressure (1 bar) of ≥ 200 °C, more preferably a boiling point at atmospheric pressure (1 bar) in the range of 200 °C to 300 °C. The method of the present invention is particularly suitable for this group of monomers **M.**

More preferably, the monomer **M** is a compound according to chemical formulae **(A)** as described above and suffices the additional requirement that it has a boiling point at atmospheric pressure (1 bar) of ≥ 200 °C, even more preferably a boiling point at atmospheric pressure (1 bar) in the range of 200 °C to 300 °C.

The gaseous stream **G_{M}** comprises the monomer **M** and is in particular essentially free of inhibitor **I**. "Essentially free of inhibitor **I**" in particular means that the content of inhibitor **I** in gaseous stream **G_{M}** is below 5 wt.-%, even more preferred below 1 wt.-%, even more preferred below 0.1 wt.-%, even more preferred below 100 ppm, even more preferred below 20 ppm.

In fact, the gist of the method according to the invention lies in the finding that the inhibitors **I**, compared to non-inventive TEMPO derivatives such as 4-OH-TEMPO or 4-Acetoxy-TEMPO, have a surprisingly low tendency to co-distill with the monomer **M**, in particular at temperatures of ≥ 200 °C, more preferably at temperatures in the range of 200 °C to 300 °C.

It is thus preferred that in step b., the liquid phase **P_{M1}** is typically heated to a temperature which corresponds to the boiling point of the monomer **M** at the applied pressure, for example to at least 200 °C, preferably to a temperature of at least 214 °C. In preferred embodiment, the pressure applied in step b. is sub-atmospheric (< 1 bar), more preferably in the range of < 500 mbar, for example in the range of from 1 mbar to 100 mbar. It goes without saying that **I** is selected so that it has a higher boiling point than the monomer **M** to be purified.

The pressure applied in step b. of the present invention is not particularly limited and preferably in the range of from 1 mbar to 1 bar, preferably in the range of from 10 mbar to 800 mbar, more preferably in the range of from 20 mbar to 500 mbar, more preferably in the range of from 20 mbar to 100 mbar.

After step b. has been carried out (and at least a part of the monomer **M** has been removed from liquid phase **P_{M1}** as gaseous stream **G_{M}**), the residual phase **P_{M2}** comprising the at least one inhibitor **I** is obtained as remaining phase **P_{M2}** (distillation sump). The ratio of the weight of all inhibitors **I** to all monomers **M** in residual phase **P_{M2}** obtained after step b. is larger than the ratio of the weight of all inhibitors **I** to all monomers **M** in the liquid phase **P_{M1}** provided in step a.

In step c. of the method according to the invention, at least a part of the gaseous stream **G_{M}** is condensed, and thus a purified liquid phase **L_{M2}** comprising **M** is obtained. The ratio of the weight of all inhibitors **I** to all monomers **M** in liquid phase **L_{M2}** obtained after step c. is smaller than the ratio of the weight of all inhibitors **I** to all monomers **M** in the liquid phase **P_{M1}** provided in step a.

The content of inhibitor **I** in liquid phase **L_{M2}** obtained after step c. is preferably below 5 wt.-%, even more preferred below 1 wt.-%, even more preferred below 0.1 wt.-%, most preferred below 0.02 wt.-%, even more preferred below 0.01 wt.-%, even more preferred below 0.001 wt.-%.

In the method according to the invention, it is preferred that the total amount of all inhibitors **I** in liquid phase **P_{M1}** provided in step a. is from 0.1 to 10000 ppm, in particular 1 to 1000 ppm, even more preferred 10 to 800 ppm, even more preferred 50 to 550 ppm, with respect to the total mass of all monomers **M** in liquid phase **P_{M1}** provided in step a.

The method according to the invention may be carried out in a typical distillation apparatus as exemplified in the example section. It is useful in any context where the monomer **M** is to be purified by distillation. For example, when this method is applied in the context of an industrial plant for the synthesis of monomers **M** or isolation of monomers **M** from gas streams, wherein the monomer **M** eventually undergoes distillation, it is preferred to add the inhibitor **I** at several points in the production plant at the same or different concentrations (for example as described in WO 2010/149527 A1, WO 2019/101296 A1).

### 2. Mixture M₁

In a second aspect of the present invention, it is provided a mixture **M₁** comprising at least one monomer **M** selected from the compounds according to one of chemical formulae **(A)**, **(B)** as defined above and at least one inhibitor **I** selected from the compounds according to chemical formulae **(I), (II), (III)** as defined above.

The monomer **M** comprised by mixture **M₁** according to the second aspect of the invention is selected from compounds according to chemical formulae **(A)**, **(B)**. Preferably, the monomer **M** comprised by mixture **M₁** is selected from compounds according to chemical formula **(A).**

Chemical formula **(A)** has the following structure:

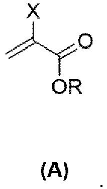

In formula **(A)**, X is selected from the group consisting of hydrogen, methyl, and is preferably hydrogen.

In formula **(A)**, R is selected from C₂ to C₂₀ alkyl, C₂ to C₂₀ hydroxyalkyl, preferably R is selected from C₄ to C₂₀ alkyl, C₂ to C₂₀ hydroxyalkyl, more preferably R is selected from C₅ to C₁₅ alkyl, C₂ to C₁₅ hydroxyalkyl, even more preferably R is C₅ to C₁₅ alkyl, even more preferably R is C₅ to C₁₂ alkyl, most preferably R = 2-ethylhexyl.

C₂ to C₂₀ hydroxyalkyl and C₂ to C₁₅ hydroxyalkyl are each preferably selected from 1-hydroxy-ethyl, 2-hydroxy-ethyl. C₂ to C₂₀ hydroxyalkyl and C₂ to C₁₅ hydroxyalkyl are each most preferably selected from 2-hydroxy-ethyl.

Chemical formula **(B)** has the following structure:

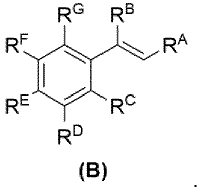

In formula **(B)**, at least one of R^{A}, R^{B}, R^{C}, R^{D}, R^{E}, R^{F}, R^{G} is selected from C₁ to C₂₀ alkyl, C₂ to C₂₀ alkenyl, and each of the other residues R^{A}, R^{B}, R^{C}, R^{D}, R^{E}, R^{F}, R^{G} is hydrogen. Preferably, in formula **(B)**, one of R^{A}, R^{B}, R^{C}, R^{D}, R^{E}, R^{F}, R^{G} is selected from C₁ to C₂₀ alkyl, C₂ to C₂₀ alkenyl, and each of the other residues R^{A}, R^{B}, R^{C}, R^{D}, R^{E}, R^{F}, R^{G} is hydrogen.

In a preferred embodiment of formula **(B)**, at least one of R^{A}, R^{B}, R^{C}, R^{D}, R^{E}, R^{F}, R^{G} is selected from C₁ to C₆ alkyl, C₂ to C₆ alkenyl, and each of the other residues R^{A}, R^{B}, R^{C}, R^{D}, R^{E}, R^{F}, R^{G} is hydrogen. Preferably, in formula **(B)**, one of R^{A}, R^{B}, R^{C}, R^{D}, R^{E}, R^{F}, R^{G} is selected from C₁ to C₆ alkyl, C₂ to C₆ alkenyl, and each of the other residues R^{A}, R^{B}, R^{C}, R^{D}, R^{E}, R^{F}, R^{G} is hydrogen.

In an even more preferred embodiment of formula **(B)**, at least one of R^{A}, R^{B}, R^{C}, R^{D}, R^{E}, R^{F}, R^{G} is selected from C₁ to C₄ alkyl, C₂ to C₄ alkenyl, and each of the other residues R^{A}, R^{B}, R^{C}, R^{D}, R^{E}, R^{F}, R^{G} is hydrogen. Preferably, in formula **(B)**, one of R^{A}, R^{B}, R^{C}, R^{D}, R^{E}, R^{F}, R^{G} is selected from C₁ to C₄ alkyl, C₂ to C₄ alkenyl, and each of the other residues R^{A}, R^{B}, R^{C}, R^{D}, R^{E}, R^{F}, R^{G} is hydrogen.

The most preferred compounds according to formula **(B)** are selected from
- α-methyl styrene (R^{B} = methyl; R^{A} = R^{C} = R^{D} = R^{E} = R^{F} = R^{G} = H);
- 1,2-divinyl benzene (R^{C} = -(CH)=CH₂; R^{A} = R^{B} = R^{D} = R^{E} = R^{F} = R^{G} = H);
- 1,3-divinyl benzene (R^{D} = -(CH)=CH₂; R^{A} = R^{B} = R^{C} = R^{E} = R^{F} = R^{G} = H);
- 1,4-divinyl benzene (R^{E} = -(CH)=CH₂; R^{A} = R^{B} = R^{G} = R^{D} = R^{F} = R^{G} = H);
- 2-tert-butyl styrene (R^{C} = tert-butyl; R^{A} = R^{B} = R^{D} = R^{E} = R^{F} = R^{G} = H);
- 3-tert-butyl styrene (R^{D} = tert-butyl; R^{A} = R^{B} = R^{C} = R^{E} = R^{F} = R^{G} = H);
- 4-tert-butyl styrene (R^{E} = tert-butyl; R^{A} = R^{B} = R^{C} = R^{D} = R^{F} = R^{G} = H);
- 2-vinyl toluene (R^{C} = -CH₃; R^{A} = R^{B} = R^{D} = R^{E} = R^{F} = R^{G} = H);
- 3-vinyl toluene (R^{D} = -CH₃; R^{A} = R^{B} = R^{C} = R^{E} = R^{F} = R^{G} = H);
- 4-vinyl toluene (R^{E} = -CH₃; R^{A} = R^{B} = R^{C} = R^{D} = R^{F} = R^{G} = H).

The most preferred monomer **M** comprised by mixture **M₁** is 2-ethylhexyl acrylate (CAS-No.: 103-11-7).

The inhibitor **I** comprised by mixture **M₁** is selected from the compounds according to chemical formulae **(I), (II), (III)**, preferably is a compound according to chemical formula **(I)** or **(II),** more preferably is a compound according to chemical formula **(I):**

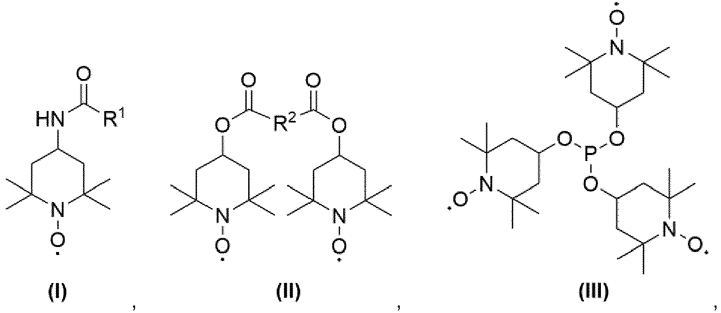

In formula **(I),** R¹ is selected from alkyl group, alkenyl group, alkynyl group, aromatic group, preferably R¹ = alkyl group, more preferably R¹ = C₁-C₂₀ alkyl group, even more preferably R¹ = C₁-C₁₅ alkyl group, even more preferably R¹ = C₁-C₁₀ alkyl group, even more preferably R¹ = C₁-C₆ alkyl group, even more preferably R¹ = C₁-C₄ alkyl group, even more preferably R¹ = C₁-C₂ alkyl group, most preferably R¹ = methyl.

In formula **(II),** R² is a divalent group selected from alkylene group, alkenylene, alkynylene, bivalent aromatic group, preferably R² = alkylene group, more preferably R² = C₁-C₂₀ alkylene group, more preferably R² = C₄-C₁₅ alkylene group, more preferably R² = C₆-C₁₂ alkylene group, more preferably R² = C₈-C₁₀ alkylene group, most preferably R² = n-octylene group.

According to the second aspect of the invention, it is preferred that the total amount of all inhibitors **I** in mixture **M₁** is from 0.1 to 10000 ppm, in particular 1 to 1000 ppm, even more preferred 10 to 800 ppm, even more preferred 50 to 550 ppm, with respect to the total mass of all monomers **M** in mixture **M₁**.

### 3. Use of at least one inhibitor I

In a third aspect according to the present invention it is provided the use of at least one inhibitor **I** selected from the compounds according to chemical formulae **(I), (II), (III)** as defined above for inhibiting the polymerization of a monomer **M** selected from the compounds according to one of chemical formulae **(A)**, **(B)** as defined in above during the purification of **M** by distillation.

The monomer **M** according to the third aspect of the invention is selected from compounds according to chemical formulae **(A)**, **(B)**. Preferably, the monomer **M** according to the third aspect of the invention is selected from compounds according to chemical formula **(A).**

Chemical formula **(A)** has the following structure:

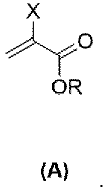

In formula **(A)**, X is selected from the group consisting of hydrogen, methyl, and is preferably hydrogen.

In formula **(A)**, R is selected from C₂ to C₂₀ alkyl, C₂ to C₂₀ hydroxyalkyl, preferably R is selected from C₄ to C₂₀ alkyl, C₂ to C₂₀ hydroxyalkyl, more preferably R is selected from C₅ to C₁₅ alkyl, C₂ to C₁₅ hydroxyalkyl, even more preferably R is C₅ to C₁₅ alkyl, even more preferably R is C₅ to C₁₂ alkyl, most preferably R = 2-ethylhexyl.

C₂ to C₂₀ hydroxyalkyl and C₂ to C₁₅ hydroxyalkyl are each preferably selected from 1-hydroxy-ethyl, 2-hydroxy-ethyl. C₂ to C₂₀ hydroxyalkyl and C₂ to C₁₅ hydroxyalkyl are each most preferably selected from 2-hydroxy-ethyl.

Chemical formula **(B)** has the following structure:

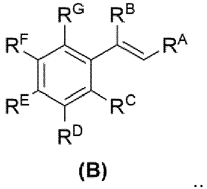

In formula **(B)**, at least one of R^{A}, R^{B}, R^{C}, R^{D}, R^{E}, R^{F}, R^{G} is selected from C₁ to C₂₀ alkyl, C₂ to C₂₀ alkenyl, and each of the other residues R^{A}, R^{B}, R^{C}, R^{D}, R^{E}, R^{F}, R^{G} is hydrogen. Preferably, in formula **(B)**, one of R^{A}, R^{B}, R^{C}, R^{D}, R^{E}, R^{F}, R^{G} is selected from C₁ to C₂₀ alkyl, C₂ to C₂₀ alkenyl, and each of the other residues R^{A}, R^{B}, R^{C}, R^{D}, R^{E}, R^{F}, R^{G} is hydrogen.

In a preferred embodiment of formula **(B)**, at least one of R^{A}, R^{B}, R^{C}, R^{D}, R^{E}, R^{F}, R^{G} is selected from C₁ to C₆ alkyl, C₂ to C₆ alkenyl, and each of the other residues R^{A}, R^{B}, R^{C}, R^{D}, R^{E}, R^{F}, R^{G} is hydrogen. Preferably, in formula **(B)**, one of R^{A}, R^{B}, R^{C}, R^{D}, R^{E}, R^{F}, R^{G} is selected from C₁ to C₆ alkyl, C₂ to C₆ alkenyl, and each of the other residues R^{A}, R^{B}, R^{C}, R^{D}, R^{E}, R^{F}, R^{G} is hydrogen.

In an even more preferred embodiment of formula **(B)**, at least one of R^{A}, R^{B}, R^{C}, R^{D}, R^{E}, R^{F}, R^{G} is selected from C₁ to C₄ alkyl, C₂ to C₄ alkenyl, and each of the other residues R^{A}, R^{B}, R^{C}, R^{D}, R^{E}, R^{F}, R^{G} is hydrogen. Preferably, in formula **(B)**, one of R^{A}, R^{B}, R^{C}, R^{D}, R^{E}, R^{F}, R^{G} is selected from C₁ to C₄ alkyl, C₂ to C₄ alkenyl, and each of the other residues R^{A}, R^{B}, R^{C}, R^{D}, R^{E}, R^{F}, R^{G} is hydrogen.

The most preferred compounds according to formula **(B)** are selected from
- α-methyl styrene (R^{B} = methyl; R^{A} = R^{C} = R^{D} = R^{E} = R^{F} = R^{G} = H);
- 1,2-divinyl benzene (R^{C} = -(CH)=CH₂; R^{A} = R^{B} = R^{D} = R^{E} = R^{F} = R^{G} = H);
- 1,3-divinyl benzene (R^{D} = -(CH)=CH₂; R^{A} = R^{B} = R^{C} = R^{E} = R^{F} = R^{G} = H);
- 1,4-divinyl benzene (R^{E} = -(CH)=CH₂; R^{A} = R^{B} = R^{C} = R^{D} = R^{F} = R^{G} = H);
- 2-*tert*-butyl styrene (R^{C} = tert-butyl; R^{A} = R^{B} = R^{D} = R^{E} = R^{F} = R^{G} = H);
- 3-*tert*-butyl styrene (R^{D} = *tert*-butyl; R^{A} = R^{B} = R^{C} = R^{E} = R^{F} = R^{G} = H);
- 4-*tert*-butyl styrene (R^{E} = *tert*-butyl; R^{A} = R^{B} = R^{C} = R^{D} = R^{F} = R^{G} = H);
- 2-vinyl toluene (R^{C} = -CH₃; R^{A} = R^{B} = R^{D} = R^{E} = R^{F} = R^{G} = H);
- 3-vinyl toluene (R^{D} = -CH₃; R^{A} = R^{B} = R^{C} = R^{E} = R^{F} = R^{G} = H);
- 4-vinyl toluene (R^{E} = -CH₃; R^{A} = R^{B} = R^{C} = R^{D} = R^{F} = R^{G} = H).

The most preferred monomer **M** in the third aspect of the invention is 2-ethylhexyl acrylate (CAS-No.: 103-11-7).

The inhibitor **I** in the third aspect of the invention is selected from the compounds according to chemical formulae **(I), (II), (III)**, preferably is a compound according to chemical formula **(I)** or **(II),** more preferably is a compound according to chemical formula **(I):**

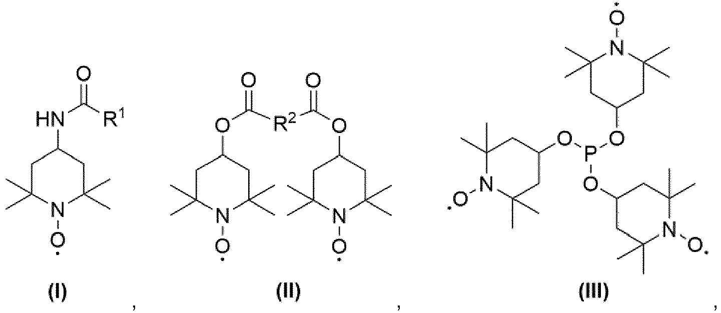

In formula **(I),** R¹ is selected from alkyl group, alkenyl group, alkynyl group, aromatic group, preferably R¹ = alkyl group, more preferably R¹ = C₁-C₂₀ alkyl group, even more preferably R¹ = C₁-C₁₅ alkyl group, even more preferably R¹ = C₁-C₁₀ alkyl group, even more preferably R¹ = C₁-C₆ alkyl group, even more preferably R¹ = C₁-C₄ alkyl group, even more preferably R¹ = C₁-C₂ alkyl group, most preferably R¹ = methyl.

In formula **(II),** R² is a divalent group selected from alkylene group, alkenylene, alkynylene, bivalent aromatic group, preferably R² = alkylene group, more preferably R² = C₁-C₂₀ alkylene group, more preferably R² = C₄-C₁₅ alkylene group, more preferably R² = C₆-C₁₂ alkylene group, more preferably R² = C₈-C₁₀ alkylene group, most preferably R² = n-octylene group.

In the third aspect of the invention, it is preferred that the total amount of all inhibitors **I** used is from 0.1 to 10000 ppm, in particular 1 to 1000 ppm, even more preferred 10 to 800 ppm, even more preferred 50 to 550 ppm, with respect to the total mass of all monomers M used.

### Examples

The tendency of different inhibitors to co-distill with the monomer 2-ethylhexyl acrylate (CAS-Nr.: 103-11-7) was tested.

The following inhibitors according to the invention were tested:
Example **I1:** 4-Acetamido-2,2,6,6-tetramethylpiperidine-1-oxyl ("4-AA-TEMPO"; CAS-Nr.: 14691-89-5).
Example **I2:** Bis(2,2,6,6-tetramethyl-1-piperidinyloxy-4-yl) sebacate ("Bis-TEMPO-sebacate"; CAS-Nr.: 2516-92-9).

The following inhibitors that are not according to the invention were tested:
Example **C1:** 4-Hydroxy-2,2,6,6-tetramethylpiperidine-1-oxyl ("4-OH-TEMPO"; CAS-Nr.: 2226-96-2).
Example **C2**: 4-Acetoxy-2,2,6,6-tetramethylpiperidine-1-oxyl ("4-Acetoxy-TEMPO"; CAS-Nr.: 6599-87-7).

### Example 11 (according to the invention)

250 g of 2-ethylhexyl acrylate containing 500 ppm 4-AA-TEMPO were initially charged at room temperature in a 1 l three necked vessel with distillation attachment (reflux condenser mounted on the distillation column) and internal thermometer. The distillation attachment included a distillation column and a flask for collecting the distillate. Thereafter, the vessel was immersed in an oil/water bath which was heated to 130 °C while an absolute pressure of 20 mbar was applied. The distillation was carried out until 80 % of the initially charged 2-ethylhexyl acrylate had distilled over (reflux ratio 5:1). The distillate was collected as three fractions of equivalent size (taken after 15 minutes, 215 minutes, 275 minutes after start of the distillation).

The content of 4-AA-TEMPO in each of the three fractions was measured.

### Example I2 (according to the invention)

The distillation according to example **I1** was repeated with 250 g of 2-ethylhexyl acrylate containing 500 ppm Bis-TEMPO-sebacate.

The content of Bis-TEMPO-sebacate in each of the three fractions was measured.

### Example C1 (comparative)

The distillation according to example **I1** was repeated with 250 g of 2-ethylhexyl acrylate containing 500 ppm 4-OH-TEMPO.

The content of 4-OH-TEMPO in each of the three fractions was measured.

### Example C2 (comparative)

The distillation according to example **I1** was repeated with 250 g of 2-ethylhexyl acrylate containing 500 ppm 4-Acetoxy-TEMPO.

The content of 4-Acetoxy-TEMPO in each of the three fractions was measured.

### Results

For each of the three fractions taken in Examples **I1**, **I2**, **C1**, and **C2**, the content of the respective inhibitor [4-AA TEMPO (in case of Example **I1**), Bis-TEMPO-sebacat (in case of Example **I2**), 4-OH-TEMPO (in case of example **C1**), 4-acetoxy TEMPO (in case of example **C2**)] was analyzed via gas chromatography equipped with a flame ionization detector (GC-FID) and electron spin resonance (ESR). The results are shown in the Figure.
1) Surprisingly, the inhibitors according to the invention displayed a much smaller general tendency to co-distill. The content of 4-AA-TEMPO and Bis-TEMPO-sebacate in all three fractions was much smaller than the content of 4-OH-TEMPO or 4-Acetoxy-TEMPO in the three fractions.
2) Even more surprisingly, in case of the comparative inhibitors 4-OH-TEMPO and 4-acetoxy-TEMPO, the amount of inhibitors in the last fraction 3 disproportionally increased compared to fraction 2 and compared to the increase betweens fractions 2 and 1.
   In contrast, such disproportionate increase was not observed for the inhibitors according to the invention, i.e. Bis-TEMPO-sebacate and 4-AA-TEMPO. Hence, there is not only a quantitative, but also a qualitative tendency of the inhibitors according to the invention to not co-distill with the monomer. Such tendency is not shown by the comparative inhibitors.

## Claims

1. Method of purifying a monomer **M** selected from the compounds according to one of chemical formulae **(A)**, **(B)** by distillation, 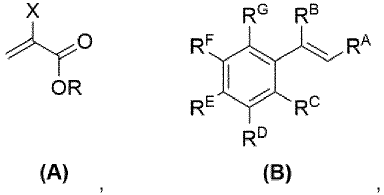
wherein X is selected from the group consisting of hydrogen, methyl,
wherein R is selected from C₂ to C₂₀ alkyl, C₂ to C₂₀ hydroxyalkyl,
wherein at least one of R^{A}, R^{B}, R^{C}, R^{D}, R^{E}, R^{F}, R^{G} is selected from C₁ to C₂₀ alkyl, C₂ to C₂₀ alkenyl, and each of the other residues R^{A}, R^{B}, R^{C}, R^{D}, R^{E}, R^{F}, R^{G} is hydrogen,
comprising the following steps:
a. providing a liquid phase **P_{M1}** comprising **M** and at least one inhibitor **I**,
b. heating **P_{M1}** to obtain a gaseous stream **G_{M}** comprising the monomer **M** and a residual phase **P_{M2}** comprising the at least one inhibitor **I**,
c. condensing at least a part of the gaseous stream **G_{M}** to obtain a purified liquid phase **L_{M2}** comprising **M**,
**characterized in that** the inhibitor **I** is selected from the compounds according to chemical formulae **(I), (II), (III):** 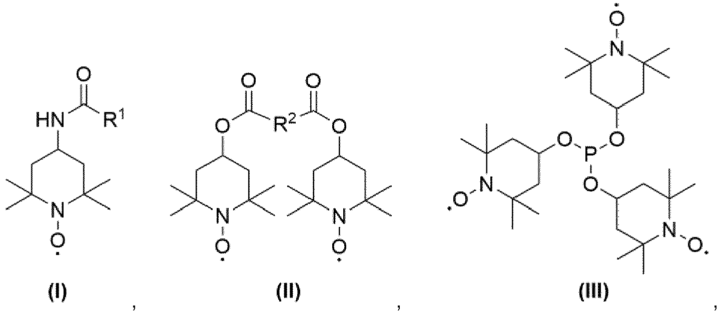
wherein R¹ is selected from alkyl group, alkenyl group, alkynyl group, aromatic group, preferably R¹ = alkyl group, more preferably R¹ = C₁-C₂₀ alkyl group;
wherein R² is a divalent group selected from alkylene group, alkenylene group, alkynylene group, bivalent aromatic group, preferably R² = alkylene group, more preferably R² = C₁-C₂₀ alkylene group.

2. Method according to Claim 1, wherein R¹ is methyl, and R² is -(CH₂)₈-.

3. Method according to Claim 1 or 2, wherein the monomer **M** is a compound according to formula **(A).**

4. Method according to Claim 3, wherein the monomer **M** is 2-ethylhexyl acrylate.

5. Method according to one of Claims 1 to 4, wherein the total amount of all inhibitors **I** in liquid phase **P_{M1}** provided in step a. is from 0.1 to 10000 ppm with respect to the total mass of all monomers **M** in liquid phase **P_{M1}** provided in step a.

6. Mixture **M₁** comprising at least one monomer **M** selected from the compounds according to one of chemical formulae **(A)**, **(B)** as defined in Claim 1 and at least one inhibitor **I** selected from the compounds according to chemical formulae **(I), (II), (III)** as defined in Claim 1.

7. Mixture **M₁** according to Claim 6, wherein R¹ is methyl, and R² is -(CH₂)₈-.

8. Mixture **M₁** according to Claim 6 or 7, wherein the monomer **M** is a compound according to formula **(A)** as defined in Claim 1.

9. Mixture **M₁** according to Claim 8, wherein the monomer **M** is 2-ethylhexyl acrylate.

10. Mixture **M₁** according to one of Claims 6 to 9, wherein the total amount of all inhibitors **I** in mixture **M₁** is from 0.1 to 10000 ppm with respect to the total mass of all monomers **M** in mixture **M₁.**

11. Use of at least one inhibitor **I** selected from the compounds according to chemical formulae **(I), (II), (III)** as defined in Claim 1 for inhibiting the polymerization of a monomer **M** selected from the compounds according to one of chemical formulae **(A), (B)** as defined in Claim 1 during the purification of **M** by distillation.

12. Use according to Claim 11, wherein R¹ is methyl, and R² is -(CH₂)₈-.

13. Use according to Claim 11 or 12, wherein the monomer **M** is a compound according to formula **(A)** as defined in Claim 1.

14. Use according to Claim 13, wherein the monomer **M** is 2-ethylhexyl acrylate.

15. Use according to one of Claims 11 to 14, wherein the inhibitor **I** is employed in such an amount so that the total amount of all inhibitors **I** is from 0.1 to 10000 ppm with respect to the total mass of all monomers **M.**
